# EUROPEAN PATENT APPLICATION

(11) **EP 4 835 898 A1**
(43) Date of publication of application: **04.11.2026**
(21) Application number: 24913825.6
(22) Date of filing: 18.12.2024
(51) Int. Cl.: G16B 45/00, G16B 50/00, C12Q 1/6844

(54) **METHOD AND APPARATUS FOR EDITING DISPLAY OF DETECTION RESULTS OF PLURALITY OF BIOLOGICAL TARGET ANALYTES**

(30) Priority: 28.12.2023 KR 20230195630
(71) Applicant: Seegene, Inc., Seoul 05548 (KR)
(72) Inventor: JOO, Jae Hyung, Yongin-si, Gyeonggi-do 16843 (KR); KIM, Sun Young, Seongnam-si, Gyeonggi-do 13568 (KR); LEE, Yoon Sang, Seoul 05625 (KR); LEE, Joonghyeok, Seoul 05547 (KR); KIM, Ki Young, Seoul 01675 (KR); CHOI, Gyusu, Seoul 05823 (KR); CHOI, Hun Seok, Seoul 05547 (KR)
(74) Representative: Viering, Jentschura & Partner mbB Patent- und Rechtsanwälte
(86) International application number: PCT/KR2024/097149
(87) International publication number: WO 2025/144021

(57) **Abstract**

The present disclosure provides a method for selectively editing presence-or-absence reading results of a plurality of target nucleic acids using a detection reagent for a multiplex nucleic acid amplification reaction. Specifically, the present disclosure provides a technique editing a plurality of target nucleic acids such that presence-or-absence reading results for some target nucleic acids are masked to hide the corresponding target nucleic acids according to a user selection so as not to be displayed, while displaying a plurality of target nucleic acids capable of being amplified using the detection reagent.

## Description

### TECHNICAL FIELD

The present disclosure relates to editing a display of detection results of a plurality of biological target analytes using a detection reagent for a multiplex nucleic acid amplification reaction.

### BACKGROUND

Molecular diagnostics is a rapidly growing field in the in vitro diagnostics market for the early diagnosis of diseases.

Among these methods, methods using nucleic acids are useful for diagnosing causative genetic factors of infections caused by a virus, bacteria, or the like, based on their high specificity and sensitivity.

Most diagnostic methods using nucleic acids use a nucleic acid amplification reaction amplifying a target nucleic acid (e.g., a viral or bacterial nucleic acid). As a representative example, polymerase chain reaction (PCR) which is one type of nucleic acid amplification reactions includes a repeated cycle process of denaturation of double-stranded DNA, annealing of an oligonucleotide primer to a DNA template, and primer extension by a DNA polymerase (Mullis et al., U.S. Patent Nos. 4,683,195, 4,683,202, and 4,800,159: Saiki et al., Science 230:1350-1354 (1985)). Other methods for amplifying nucleic acids include ligase chain reaction (LCR) (U.S. Patent Nos. 4,683,195 and 4,683,202; PCR Protocols: A Guide to Methods and Applications (Innis et al., eds, 1990)), strand displacement amplification (SDA) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2):35-40 (1999)), Q-Beta Replicase (Lizardi et al., BiolTechnology 6:1197 (1988)), Loop-mediated isothermal amplification (LAMP, Y. Mori, H. Kanda and T. Notomi, J. Infect. Chemother., 2013, 19, 404-411), and Recombinase polymerase amplification (RPA, J. Li, J. Macdonald and F. von Stetten, Analyst, 2018, 144, 31-67).

Recently, multiplex diagnostic technology detecting a plurality of target nucleic acids in a single tube based on such a nucleic acid amplification reaction has been used. For example, various multiplex technologies exist for detecting various types of viruses at once using methods including the PCR and LAMP described above as examples of the nucleic acid amplification reaction.

Multiplex nucleic acid amplification reaction technologies require the use of a detection reagent for a multiplex nucleic acid amplification reaction including oligonucleotides (e.g., primers and/or probes) specifically hybridizing to a target nucleic acid, a label, a DNA polymerase, dNTPs, Mg ions, a buffer, and the like to amplify and detect the target nucleic acid of interest.

Since such a detection reagent for a multiplex nucleic acid amplification reaction may simultaneously amplify a plurality of target nucleic acids in a single reaction through a multiplex technique, an analysis target capable of being amplified is set according to the characteristics of the detection reagent.

The detection reagent for a multiplex nucleic acid amplification reaction is commercialized as a diagnostic kit together with other components related to an amplification reaction, and a patient may select a diagnostic kit according to a suspected disease, and may be diagnosed with an infection by detecting at least one target nucleic acid through an amplification reaction using a detection reagent of the selected diagnostic kit.

In this way, in the case of a detection reagent using a multiplex technique, since various target nucleic acids may be simultaneously amplified through a single test, the presence or absence of a plurality of corresponding target nucleic acids may be checked according to the characteristics of the detection reagent.

However, depending on the patient, only the presence or absence of a certain virus, that is, a specific target nucleic acid, among a plurality of target nucleic acids may be of interest. This is because checking only the presence or absence of a virus clearly suspected of causing a disease may be practical for each patient in terms of unnecessary medical practices and costs.

Nevertheless, when using a diagnostic kit to which a multiplex technique is applied, an analysis target of a corresponding detection reagent, that is, the presence or absence of each of a plurality of amplifiable target nucleic acids, is simultaneously and collectively checked, so that medical practices are performed or costs are incurred for the presence or absence of target nucleic acids not of interest.

### SUMMARY

A problem to be solved according to an embodiment is to provide a method for selectively editing presence-or-absence reading results of a plurality of target nucleic acids using the aforementioned detection reagent.

Specifically, providing a technique for editing a plurality of target nucleic acids such that presence-or-absence reading results for some target nucleic acids are masked to hide the corresponding target nucleic acids according to a user selection so as not to be displayed, while displaying a plurality of target nucleic acids capable of being amplified using a detection reagent for a multiplex nucleic acid amplification reaction, may be included in this task.

However, the problem to be solved by the present disclosure is not limited to those mentioned above, and other unmentioned problems to be solved will be clearly understood by those skilled in the art to which the present disclosure pertains from the following description.

In a computer-implemented method for editing a display of detection results of a plurality of biological target analytes to be performed by a detection result display editing apparatus according to the present disclosure, the method comprises accessing, by the detection result display editing apparatus, plate setup information for a reaction plate on which an amplification reaction is to be performed using a detection reagent for a multiplex nucleic acid amplification reaction capable of detecting the plurality of biological target analytes, wherein the detection result display editing apparatus includes (i) a memory, (ii) a processor, and (iii) one or more programs stored in the memory and executable by the processor, wherein the plate setup information comprises setup information for each of a plurality of reaction wells, and wherein the setup information for each of the plurality of reaction wells comprises identifiers for the plurality of biological target analytes amplified in that reaction well; displaying the identifiers on a masking data input screen of the detection result display editing apparatus; receiving editing information that determines whether to mask an amplification reaction result in a detection result display apparatus for an identifier of at least one biological target analyte among the identifiers for the plurality of biological target analytes amplified in that reaction well through the masking data input screen; and editing the received plate setup information according to the editing information and providing the edited plate setup information.

Furthermore, the accessing operation may include accessing the plate setup information i) stored in the detection result display apparatus or an external apparatus or ii) stored in the detection result display editing apparatus, and the plate setup information stored in the detection result display editing apparatus is received from the detection result display apparatus or the external apparatus.

Furthermore, the identifiers may be names of the plurality of biological target analytes to be amplified using the detection reagent, and when the detection reagent includes a plurality of detection reagents, identifiers of a target analyte for the plurality of detection reagents may be displayed on the masking data input screen according to each of the plurality of detection reagents.

Furthermore, the displaying operation may include displaying the identifier for each of the plurality of biological target analytes as objects individually selectable by a user.

Furthermore, the displaying operation may include distinctively displaying at least one object selected by the user and at least one unselected object among the objects displayed on the masking data input screen.

Furthermore, for the selected object, masking of a corresponding identifier and the amplification reaction result may be determined in the detection result display apparatus, and for the unselected object, the corresponding identifier and the amplification reaction result may be displayed in the detection result display apparatus.

Furthermore, the editing information may be generated using information indicating whether the individually selectable object is selected based on the accessed plate setup information.

Furthermore, the plate setup information stored in the detection result display editing apparatus may be further edited according to a template in which an identifier to be masked among the plurality of biological target analytes capable of being detected is predefined based on the detection reagent.

Furthermore, the template may be preset editing information, and whether the amplification reaction result is masked for an identifier of at least one preset biological target analyte among identifiers for the plurality of biological target analytes may be stored in the preset editing information.

Furthermore, the template may be editing information masking remaining target analytes except for a target analyte preferentially suspected by time, symptom, and age group.

Furthermore, the template may be classified based on at least one of time elapsed since symptom onset, symptom, and/or gender, and age group.

Furthermore, the time elapsed since symptom onset may be 1 hour, 2 hours, 3 hours, 4 hours, 5 hours, 6 hours, 7 hours, 8 hours, 9 hours, 10 hours or more, or 96 hours, 72 hours, 48 hours, 36 hours, 24 hours or less.

Furthermore, the symptom may be any one of a type of symptom, a mild symptom, and a severe symptom.

Furthermore, the method may further include exporting the plate setup information further edited according to the template to the detection result display apparatus and/or the external storage apparatus.

Furthermore, when the plate setup information may be stored in the detection result display apparatus or the external apparatus, the method may further comprise exporting the editing information to the detection result display apparatus and/or the external storage apparatus.

Furthermore, in the exporting operation, the editing information on whether the identifiers of the plurality of biological target analytes are masked may be converted into a hexadecimal number and exported.

A detection result display editing apparatus for editing a display of detection results of a plurality of biological target analytes may comprise a memory, a processor, and one or more programs stored in the memory and executable by the processor. When executed by the processor, the one or more programs may cause the detection result display editing apparatus to access plate setup information for a reaction plate on which an amplification reaction is to be performed using a detection reagent for a multiplex nucleic acid amplification reaction capable of detecting the plurality of biological target analytes, wherein the plate setup information comprises setup information for each of a plurality of reaction wells, and the setup information for each of the plurality of reaction wells comprises identifiers for the plurality of biological target analytes amplified in that reaction well; display the identifiers on a masking data input screen of the detection result display editing apparatus; receive editing information that determines whether to mask an amplification reaction result in a detection result display apparatus for an identifier of at least one biological target analyte among the identifiers for the plurality of biological target analytes amplified in that reaction well through the masking data input screen; and edit the received plate setup information according to the editing information and provide the edited plate setup information.

A non-transitory computer-readable storage medium may store a computer program executable by one or more processors included in a detection result display editing apparatus. When executed by the one or more processors, the computer program may cause the detection result display editing apparatus to perform a method including accessing, by the detection result display editing apparatus, plate setup information for a reaction plate on which an amplification reaction is to be performed using a detection reagent for a multiplex nucleic acid amplification reaction capable of detecting the plurality of biological target analytes, wherein the plate setup information comprises setup information for each of a plurality of reaction wells, and the setup information for each of the plurality of reaction wells comprises identifiers for the plurality of biological target analytes amplified in that reaction well; displaying the identifiers on a masking data input screen of the detection result display editing apparatus; receiving editing information that determines whether to mask an amplification reaction result in a detection result display apparatus for an identifier of at least one biological target analyte among the identifiers for the plurality of biological target analytes amplified in that reaction well through the masking data input screen; and editing the received plate setup information according to the editing information and providing the edited plate setup information.

According to the present disclosure, practical diagnosis in terms of unnecessary medical practices and costs may be achieved by checking only the presence or absence of a virus clearly suspected of causing a disease.

According to another embodiment, quick diagnosis and prescription are possible by first checking a causative pathogen preferentially suspected according to symptom, age, and incubation time.

### BRIEF DESCRIPTION OF THE DRAWINGS

FIG. 1 illustrates a detection result display editing apparatus for a plurality of biological target analytes and apparatuses connected thereto according to the present disclosure.
FIG. 2 is a block diagram of a detection result display editing apparatus for a plurality of biological target analytes according to the present disclosure.
FIG. 3 is a flowchart of a method for editing a display of detection results of a plurality of biological target analytes according to the present disclosure.
FIG. 4 is an implementation example of a masking data input screen of the detection result display editing apparatus (100).
FIG. 5 is an implementation example of a screen displaying amplification reaction results for all target analytes before masking an identifier for a target analyte using the detection result display editing apparatus (100) of the present disclosure or before using the detection result display editing apparatus (100).
FIG. 6 is an implementation example of a screen displaying an identifier masked in the detection result display editing apparatus (100) according to the present disclosure as amplification reaction result data in the detection result display apparatus (300).

### DETAILED DESCRIPTION OF THE INVENTION

Advantages and features of the present disclosure, and methods of achieving them will become apparent with reference to the embodiments described below in detail in conjunction with the accompanying drawings. However, the present disclosure is not limited to the embodiments disclosed below, but may be implemented in various different forms. The present embodiments are merely provided to complete the disclosure of the present disclosure and to fully inform those of ordinary skill in the art to which the present disclosure pertains of the scope of the disclosure, and the present disclosure is only defined by the scope of the claims.

In describing the embodiments of the present disclosure, when determining a detailed description of a known function or configuration may unnecessarily obscure the subject matter of the present disclosure, the detailed description thereof will be omitted. Terms described below are defined considering functions in the embodiments of the present disclosure, and may vary depending on an intention or custom of a user or an operator. Therefore, the definition should be made based on the contents throughout the present specification.

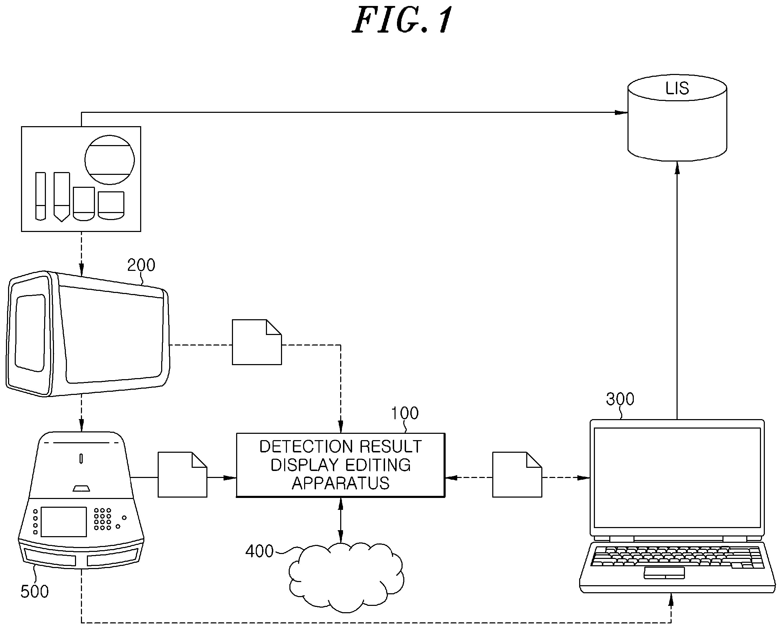

Before describing FIG. 1, terms used herein will be looked at.

The term "target analyte" includes various substances (e.g., biological substances and non-biological substances), referring to the same object as the term "target analyte".

Such a target analyte may specifically include a biological substance, more specifically, at least one of a nucleic acid molecule (e.g., DNA and RNA), a protein, a peptide, a carbohydrate, a lipid, an amino acid, a biological compound, a hormone, an antibody, an antigen, a metabolite, and a cell.

The term "sample" collectively refers to a biological sample (e.g., a cell, a tissue, and a body fluid) and a non-biological sample (e.g., food, water, and soil). Among them, the biological sample may include, for example, at least one of a virus, a bacterium, a tissue, a cell, blood (including whole blood, plasma, and serum), lymph, bone marrow fluid, saliva, sputum, a swab, an aspiration, milk, urine, a stool, ocular fluid, semen, a brain extract, spinal fluid, joint fluid, thymus fluid, bronchial wash fluid, ascites, and amniotic fluid. Such a sample may or may not include the aforementioned target analyte.

Meanwhile, when the aforementioned target analyte is a nucleic acid molecule or includes a nucleic acid molecule, a nucleic acid extraction process known in the art may be performed on the sample estimated to include the target analyte (Reference: Sambrook, J. et al., Molecular Cloning. A Laboratory Manual, 3^{rd} ed. Cold Spring Harbor Press (2001)). The nucleic acid extraction process may vary depending on a type of the sample. Furthermore, when the extracted nucleic acid is an RNA, a reverse transcription process synthesizing a cDNA may be additionally performed (Reference: Sambrook, J. et al., Molecular Cloning. A Laboratory Manual, 3rd ed. Cold Spring Harbor Press (2001)).

The term "dataset" refers to data obtained from a signal generating reaction for the target analyte using a signal generating means (the signal generating means will be described later).

In this case, the term "signal generating reaction" means a reaction generating a signal depending on properties, for example, activity, amount, or presence (or absence), specifically, presence (or absence), of the target analyte in a sample. Such a signal generating reaction includes a biological reaction and a chemical reaction. Among them, the biological reaction includes a genetic analysis process such as PCR, real-time PCR, isothermal amplification, and microarray analysis, an immunological analysis process, and a bacterial growth analysis. In addition, the chemical reaction includes a process analyzing generation, change, or destruction of a chemical substance. According to the present disclosure, the signal generating reaction may be a genetic analysis process, or may be a nucleic acid amplification reaction, an enzyme reaction, or microbial growth.

Meanwhile, the aforementioned signal generating reaction accompanies a signal change. Therefore, a progress degree of such a signal generating reaction may be evaluated by measuring the signal change.

Here, the term "signal" means a measurable output. In addition, the measured magnitude or change of such a signal serves as an indicator qualitatively or quantitatively indicating the properties of the target analyte, specifically, the presence or absence of the target analyte in the sample.

Here, an example of the indicator includes fluorescence intensity, luminescence intensity, chemiluminescence intensity, bioluminescence intensity, phosphorescence intensity, charge transfer, voltage, current, power, energy, temperature, viscosity, light scatter, radioactivity intensity, reflectivity, transmittance, and absorbance, but is not limited thereto.

The aforementioned term "signal generating means" means a means providing a signal indicating the properties, specifically, presence or absence, of the target analyte to be analyzed.

Such a signal generating means includes a label itself or an oligonucleotide to which the label connects.

Among them, the label includes a fluorescent label, a luminescent label, a chemiluminescent label, an electrochemical label, and a metal label. The label may be used as the label itself, such as an intercalating dye. Alternatively, the label may be used in a form connected to one or more oligonucleotides as a single label or an interactive dual label including a donor molecule and an acceptor molecule.

When using a fluorescent label, a signal value may be expressed as an RFU (Relative Fluorescence Unit) value.

The signal generating means may additionally include an enzyme having nucleic acid cleavage activity to generate a signal (e.g., an enzyme having 5' nucleic acid cleavage activity or an enzyme having 3' nucleic acid cleavage activity).

Meanwhile, various methods generating a signal indicating the presence of a target analyte, particularly a target nucleic acid molecule, using the signal generating means are known. A representative example may include the following: a TaqMan^{™} probe method (U.S. Patent No. 5,210,015), a molecular beacon method (Tyagi, Nature Biotechnology, v.14 MARCH 1996), a Scorpion method (Whitcombe et al., Nature Biotechnology 17:804-807 (1999)), a Sunrise (Sunrise or Amplifluor) method (Nazarenko et al., Nucleic Acids Research, 25(12):2516-2521 (1997), and U.S. Patent No. 6,117,635), a Lux method (U.S. Patent No. 7,537,886), CPT (Duck P, et al. Biotechniques, 9:142-148 (1990)), an LNA method (U.S. Patent No. 6,977,295), a Plexor method (Sherrill CB, et al., Journal of the American Chemical Society, 126:4550-4556 (2004)), Hybeacons (D.J. French, et al., Molecular and Cellular Probes 13:363-374 (2001) and U.S. Patent No. 7,348,141), a dual-labeled, self-quenched probe (U.S. Patent No. 5,876,930), a hybridization probe (Bernard PS, et al., Clin Chem 2000, 46, 147-148), a PTOCE (PTO cleavage and extension) method (WO 2012/096523), a PCE-SH (PTO Cleavage and Extension-Dependent Signaling Oligonucleotide Hybridization) method (WO2013/115442), a PCE-NH (PTO Cleavage and Extension-Dependent Non-Hybridization) method (PCT/KR2013/012312), and a CER method (WO 2011/037306).

Meanwhile, the aforementioned term "signal generating reaction" may include an amplification reaction of a signal. At this time, the term "amplification reaction" means a reaction increasing or decreasing the signal generated by the signal generating means. Specifically, the amplification reaction means an increasing (or amplifying) reaction of the signal generated by the signal generating means depending on the presence of the target analyte.

In such an amplification reaction, amplification of the target analyte (e.g., a nucleic acid molecule) may or may not be accompanied. More specifically, the amplification reaction may mean an amplification reaction of a signal accompanying amplification of the target analyte.

Meanwhile, a dataset obtained through the amplification reaction may include an amplification cycle.

Here, the term "cycle" refers to a unit of change in conditions in a plurality of measurements accompanying a change in constant conditions. The change in constant conditions means, for example, an increase or decrease in temperature, reaction time, number of reactions, concentration, pH, number of replications of a measurement target (e.g., a nucleic acid), and the like. Therefore, the cycle may be a time or process cycle, a unit operation cycle, and a reproductive cycle.

More specifically, the term "cycle" means one unit of the repetition when repeating a reaction of a constant process or repeating a reaction based on a constant time interval.

Alternatively, the term "cycle" may mean one unit of the repetition when a constant action repeats according to the progress of a reaction.

For example, when a nucleic acid amplification reaction is performed, an action detecting a signal generated at regular time intervals may be repeated, and may mean one unit of the repetition. In this case, the cycle may have a unit of time.

For example, in a case of a nucleic acid amplification reaction, one cycle means a reaction including a denaturation operation of a nucleic acid, an annealing operation of a primer, and an extension operation of a primer. In this case, the change in constant conditions is an increase in the number of repetitions of the reaction, and a repetition unit of the reaction including the series of operations is set as one cycle. The number of cycles may include the number of reactions or reaction time.

Meanwhile, the aforementioned target analyte or target analyte, particularly a target nucleic acid molecule, may be amplified by various methods: polymerase chain reaction (PCR), ligase chain reaction (LCR) (U.S. Patent Nos. 4,683,195 and 4,683,202; PCR Protocols: A Guide to Methods and Applications (Innis et al., eds, 1990)), strand displacement amplification (SDA) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2):35-40 (1999)) and Q-beta Replicase (Lizardi et al., BiolTechnology 6:1197 (1988)), Loop-mediated isothermal amplification (LAMP, Y. Mori, H. Kanda and T. Notomi, J. Infect. Chemother., 2013, 19, 404-411), Recombinase polymerase amplification (RPA, J. Li, J. Macdonald and F. von Stetten, Analyst, 2018, 144, 31-67).

Meanwhile, the amplification reaction amplifies a signal while accompanying amplification of the target analyte (specifically, the target nucleic acid molecule). For example, the amplification reaction is performed according to PCR, specifically real-time PCR, or is performed by an isothermal amplification reaction (e.g., LAMP or RPA).

Meanwhile, a dataset obtained by the signal generating reaction includes a plurality of data points including cycles of the signal generating reaction and signal values in the cycles.

Here, the term "signal value" means a value obtained by quantifying a level of a signal (e.g., intensity of a signal) actually measured in a cycle of a signal generating reaction, particularly an amplification reaction, according to a constant scale, or a modified value thereof. The modified value may include a mathematically processed signal value of the actually measured signal value. An example of the mathematically processed signal value of the actually measured signal value (that is, a signal value of a raw dataset) may include a logarithm value or a derivative value.

The term "data point" means a coordinate value including a cycle and a signal value. In addition, the term "data" means all information constituting the dataset. For example, each of the cycle and the signal value of the amplification reaction may correspond to data.

Data points obtained by a signal generating reaction, particularly an amplification reaction, may be displayed as coordinate values displayable in a two-dimensional Cartesian coordinate system. In the coordinate values, an X-axis represents a corresponding cycle number, and a Y-axis represents a signal value measured or processed in the corresponding cycle.

The term "dataset" means a set of the data points. For example, the dataset may be a set of data points directly obtained through an amplification reaction performed in the presence of a signal generating means or may be a modified dataset modifying such a dataset. The dataset may be a part or all of a plurality of data points obtained by an amplification reaction or modified data points thereof.

Meanwhile, the dataset may be a dataset obtained by processing a plurality of datasets. When an analysis on a plurality of target analytes performs in a single reaction well, a dataset for the plurality of target analytes may be obtained through processing of datasets obtained from a reaction performed in the single reaction well depending on a case. For example, a dataset for a plurality of target analytes performed in a single reaction well may be obtained by processing a plurality of datasets obtained from signals measured at different temperatures.

The aforementioned dataset may be plotted, whereby an amplification curve may be obtained.

The term "plate" refers to a standard unit in which an amplification reaction is performed in an amplification apparatus, and means a basic unit in which data generated after the amplification reaction is stored. Different plates may be plates on which amplification reactions are performed at different times using the same amplification apparatus, or may be plates on which amplification reactions are performed by different amplification apparatuses at the same time.

The plate includes a plurality of reaction wells, that is, reaction vessels. The plate may include N x M reaction wells. Typically, the plate includes 12 x 8 or 8 x 12 reaction wells. Each of the plurality of reaction wells of the plate may be integrated with the plate or may be in a separable tube form. The plate may have a rectangular shape, but the plate includes one or more reaction wells and may be implemented in various shapes such as a circle, a ladder, and a rhombus in addition to the rectangle.

The well of the plate includes a sample to be analyzed and a detection reagent for a multiplex nucleic acid amplification reaction required for a nucleic acid amplification reaction. Hereinafter, various embodiments of the present disclosure will be looked at with reference to the drawings.

FIG. 1 illustrates a detection result display editing apparatus 100 for a plurality of biological target analytes and a nucleic acid preparation apparatus 200, a detection result display apparatus 300, and a cloud server 400 connected thereto according to the present disclosure. These apparatuses 100, 200, 300, and 400 may be connected to each other by wired or wireless communication. However, FIG. 1 is merely an example, and the spirit of the present disclosure is not limitedly interpreted to the one illustrated in FIG. 1. In one embodiment, a configuration may be additionally connected to these apparatuses 100, 200, 300, and 400, which may include a nucleic acid detection apparatus 500 and/or a barcode recognition apparatus (not shown) and/or a connection apparatus (not shown) generating setup information for a reaction plate to be an amplification reaction target in the nucleic acid detection apparatus 500 and transmitting the setup information to the detection result display editing apparatus 100, but is not limited thereto.

Meanwhile, differently from the illustration, the detection result display editing apparatus 100 for a plurality of biological target analytes (hereinafter, the detection result display editing apparatus) may be implemented by being included in the detection result display apparatus 300. However, hereinafter, descriptions will be made on the premise that the aforementioned configurations 100, 200, 300, 400, and 500 are implemented or connected as illustrated in FIG. 1. Hereinafter, each configuration will be looked at in detail.

First, the nucleic acid preparation apparatus 200 may use a commonly known automated preparation apparatus (e.g., Liquid Handling Instruments), extract different nucleic acids from various types of biological samples including a nucleic acid, and prepare a reaction solution for amplification using the extracted nucleic acid.

The nucleic acid preparation apparatus 200 is an automated liquid handling apparatus.

The automated liquid handling apparatus may automatically and programmatically aspirate and/or dispense a desired amount of reagent, sample, or other liquid from a designated container for automation of a chemical or biochemical laboratory. Various configurations of an automated liquid handling apparatus are known to those skilled in the art.

For example, an apparatus by Hamilton company in Switzerland. For example, Hamilton STAR, Hamilton NIMBUS, Hamilton STARplus, and Hamilton STARlet. All parts of such a nucleic acid preparation apparatus 200 are designed as an integrated apparatus and located within a housing.

In one embodiment, the nucleic acid preparation apparatus 200 may include a nucleic acid extraction reagent arranging unit, a detection reagent for nucleic acid amplification arranging unit, a specimen arranging unit, a nucleic acid extracting unit, a nucleic acid extract arranging unit, a reaction solution for nucleic acid amplification preparing unit, and a pipetting unit.

Furthermore, the nucleic acid preparation apparatus 200 may include an extraction reagent cartridge containing various biological samples including a nucleic acid and buffers used to extract the nucleic acid therefrom, a detection reagent for nucleic acid amplification cartridge preparing a reaction solution for amplification for an amplification reaction, a cold block, a hot block, a waste liquid container, a pipetting block, a pipettor cartridge, and a pipettor, and these apparatuses may be mounted and detached in the nucleic acid preparation apparatus 200.

The pipetting block may include one or more pipettors moving and operating independently or dependently. A tip or a needle is coupled to an end of the pipettor and used to aspirate and dispense a solution.

The nucleic acid preparation apparatus 200 preparing a nucleic acid amplification reaction by extracting a nucleic acid from a biological sample containing a nucleic acid may add a barcode recognition apparatus. Biological sample information recognized by the barcode recognition apparatus may be databased and stored in a real-time medical server cloud, a Laboratory Information Management System (LIMS), and a Laboratory Information System (LIS), and such information may be automatically applied to a real-time nucleic acid detection apparatus. The barcode represents contents capable of identifying between samples, such as a gender, age, and type of a biological sample, and classifying the sample based on necessary contents.

Through such a configuration, preparation operations performed by the nucleic acid preparation apparatus 200 include a nucleic acid extract preparation operation or a reaction solution for nucleic acid amplification preparation operation, or a preparation operation simultaneously performing a nucleic acid extract preparation operation and a reaction solution for nucleic acid amplification preparation operation, but is not limited thereto.

These preparation operations are for preparing a reaction plate to be a target of an amplification reaction in a nucleic acid detection apparatus by extracting a nucleic acid, dispensing the extracted nucleic acid into a reaction well included in a plate, and dispensing a reagent typically required for an amplification reaction into each of the plurality of reaction wells into which the nucleic acid is dispensed. The reaction plate includes a plurality of wells (reaction vessels), and a sample to be analyzed (extracted nucleic acid, that is, patient specimen) and a detection reagent for a multiplex nucleic acid amplification reaction typically required for a nucleic acid amplification reaction are accommodated in the well.

When the preparation operations are driven, setup information for a plate to be an amplification reaction target in the nucleic acid detection apparatus 500 is generated in the nucleic acid preparation apparatus 200.

The reaction plate includes a plurality of reaction wells, and each of a plurality of reaction wells is arranged at different locations on the reaction plate, so that different specimens and different detection reagents may be accommodated. For the specimen accommodated in each of a plurality of reaction wells, a positive or negative reading may be read according to a result of an amplification reaction to determine a presence or absence of a disease of a patient.

Furthermore, specific target analytes capable of being amplified may be different depending on a type of a detection reagent, and amplification reaction conditions in a nucleic acid detection apparatus performing an amplification reaction may also be different for each of the plurality of detection reagents, so that the information needs to be clearly identified, stored, and shared in an environment detecting and analyzing a target analyte.

For this purpose, the present disclosure generates setup information for a plate to be an amplification reaction target inputted in a process of each preparation operation when a nucleic acid extract preparation operation and/or a reaction solution for nucleic acid amplification preparation operation is driven in the nucleic acid preparation apparatus 200.

Herein, the plate setup information may include amplification reaction protocol information, setup information for each of a plurality of reaction wells (e.g., detection channel information for each of a plurality of reaction wells, sample type), extraction reagent and detection reagent information, a type of the reaction plate, a type of each of the plurality of reaction wells, patient identification information, and patient identification information for each reaction vessel.

Looking at a specific form of the plate setup information as an example, a file having a file name of '.plrn' is generated, and the generated file may be stored in a specific location (a medical service cloud server and/or a Hospital Information System (HIS)) and may be exported to the detection result display editing apparatus 100.

The detection result display editing apparatus 100 according to the present disclosure is an apparatus using a memory, a processor, and one or more programs stored in the memory and executable by the processor, and particularly for editing a display of detection results of a plurality of biological target analytes in the detection result display apparatus 300.

Hereinafter, the detection result display editing apparatus 100 will be looked at more specifically.

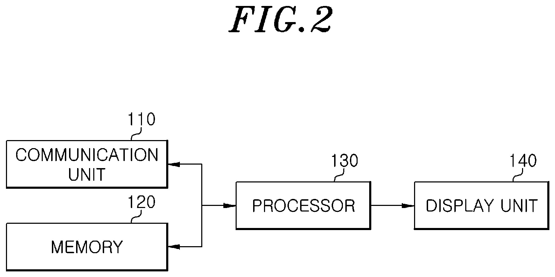

FIG. 2 is a block diagram of the detection result display editing apparatus 100 according to the present disclosure.

Referring to FIG. 2, the detection result display editing apparatus 100 includes a communication unit 110, a memory 120, a processor 130, and a display unit 140, but is not limited thereto.

First, the communication unit 110 is implemented as a wired or wireless communication module. The detection result display editing apparatus 100 may perform communication with the outside through such a communication unit 110. In one embodiment, the detection result display editing apparatus 100 may receive amplification result data from the nucleic acid preparation apparatus 200 through the communication unit 110. Furthermore, the detection result display editing apparatus 100 may receive information necessary for editing a detection result display from the outside, for example, the apparatuses 200, 300, 400, and 500, and LIS illustrated in FIG. 1 and/or a medical information system including LIMS and HIS not illustrated in FIG. 1.

The memory 120 stores various types of data or information including at least one instruction. As data to be stored, data received from external apparatuses 200, 300, 400, and 500, and LIS through the communication unit 110 or data processed by the processor 130 may be stored, and various types of editing information and the like necessary for editing a detection result display may be included in the data or information.

Meanwhile, although the memory 120 is illustrated as a separate configuration from the processor 130 in FIG. 2, the memory 120 may be implemented as a single apparatus with the processor 130. In one embodiment, the memory 120 may be a storage such as a cache included inside the processor 130.

Next, the processor 130 may be implemented by a central processing unit (CPU), a graphics processing unit (GPU), a micro controller unit (MCU), or a dedicated processor performing methods according to embodiments. Hereinafter, such a processor 130 may collectively refer to a single processor or a plurality of processors, for example, a multi-core processor.

Such a processor 130 may write data to the memory 120. Furthermore, the processor 130 may read and execute an instruction stored in the memory 120. In one embodiment, the processor 130 may cause a detection result display editing apparatus 100 to perform functions described below by executing an instruction stored in the memory 120, and such functions will be described later.

The display unit 140 may be driven by the processor 130 as follows.

First, information may be displayed on such a display unit 140. Furthermore, a user may input predefined information through the display unit 140. For such a display or input, the display unit 140 may be implemented as a touch screen or a touch pad, or alternatively, may be implemented through a combination of an LCD monitor and a keyboard. Herein, information displayed on the display unit 140 may be received from the outside by the detection result display editing apparatus 100 through the communication unit 110 or may be loaded from the memory 120, but is not limited thereto.

Depending on an embodiment, a masking data input screen 40 described later receiving detection result display editing information may be displayed on the display unit 140. Contents or materials included in such a screen may be received from the outside by the detection result display editing apparatus 100 through the communication unit 110 or loaded from the memory 120, but is not limited thereto.

Detection result display editing information inputted to the display unit 140 may be generated by a user input action through the masking data input screen 40.

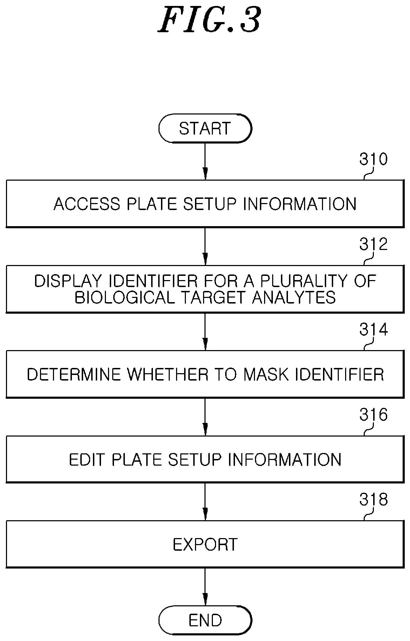

Hereinafter, each functional configuration of the detection result display editing apparatus 100 providing such a masking data input screen 40 will be looked at more specifically with reference to FIGS. 3 to 6.

According to the present disclosure, the detection result display editing apparatus 100 may access plate setup information for a reaction plate on which an amplification reaction is to be performed using a detection reagent for a multiplex nucleic acid amplification reaction capable of detecting a plurality of biological target analytes.

Depending on an embodiment, accessing in the detection result display editing apparatus 100 means accessing the plate setup information i) stored in the detection result display apparatus or an external apparatus or ii) stored in the detection result display editing apparatus, and the plate setup information stored in the detection result display editing apparatus 100 may be received from the detection result display apparatus 300 or the external apparatus.

Furthermore, depending on an embodiment, accessing the plate setup information in the detection result display editing apparatus 100 means an action receiving plate setup information from the outside, or reading plate setup information by approaching an external apparatus, or bringing plate setup information stored in the detection result display editing apparatus 100. At this time, the outside of the detection result display editing apparatus 100 may be the nucleic acid preparation apparatus 200, the detection result display apparatus 300, and a medical service cloud server 400 according to the present disclosure, but is not limited thereto, and may include all apparatuses existing outside/inside the detection result display editing apparatus 100. Specifically, accessing means approaching data generated inside based on data stored in the detection result display apparatus 300 and/or data stored in the detection result display editing apparatus 100 and/or data stored outside the detection result display editing apparatus 100 and the detection result display apparatus 300 and/or at least one piece of data received from the outside or the detection result display apparatus 300.

The accessed plate setup information comprises setup information for each of a plurality of reaction wells, and the setup information for each of the plurality of reaction wells comprises identifiers for the plurality of biological target analytes amplified in that reaction well.

The detection result display editing apparatus 100 according to the present disclosure may display the identifiers on a masking data input screen of the detection result display editing apparatus 100.

At this time, the identifier means a respective name of a plurality of biological target analytes capable of being amplified by a corresponding detection reagent using the detection reagent.

When the detection reagent includes a plurality of detection reagents, an identifier of a target analyte for the plurality of detection reagents may be displayed on the masking data input screen according to each of the plurality of detection reagents.

Nucleic acid amplification reaction technologies require use of a detection reagent for a multiplex nucleic acid amplification reaction including oligonucleotides (e.g., primers and/or probes) specifically hybridizing to a target analyte, a label, a DNA polymerase, dNTPs, Mg ions, a buffer, and the like to amplify and detect the target analyte of interest.

Such a detection reagent may be commercialized in a form of a diagnostic kit together with other components related to an amplification reaction, for example, Seegene's Allplex^{TM} Respiratory Panel1 diagnostic kit sets Influenza A virus (Flu A), Influenza A virus A-H1 (Flu A-H1), Influenza A virus A-H3 (Flu A-H3), Respiratory syncytial virus A (RSV A), and Respiratory syncytial virus B (RSV B) as amplifiable analysis targets to check an infection of Respiratory Infections.

Therefore, when a patient specimen, for example, a Nasopharyngeal swab or Nasopharyngeal aspirate is dispensed into a reaction well together with a nucleic acid extraction reagent through the nucleic acid preparation apparatus 200 to extract a nucleic acid, and the extracted nucleic acid performs an amplification reaction through a nucleic acid detection apparatus using the Allplex^{TM} Respiratory Panel1 diagnostic kit, an infection of at least one of Influenza A virus (Flu A), Influenza A virus A-H1 (Flu A-H1), Influenza A virus A-H3 (Flu A-H3), Respiratory syncytial virus A (RSV A), and Respiratory syncytial virus B (RSV B) set as analysis targets in the Allplex^{TM} Respiratory Panel1 diagnostic kit may be known through detection.

At this time, the analysis target means the identifier according to the present disclosure.

Such a detection reagent is for a multiplex amplification reaction capable of simultaneously amplifying a plurality of biological target analytes, and target analytes for each of the plurality of detection reagents, that is, analysis targets thereof may be different from each other.

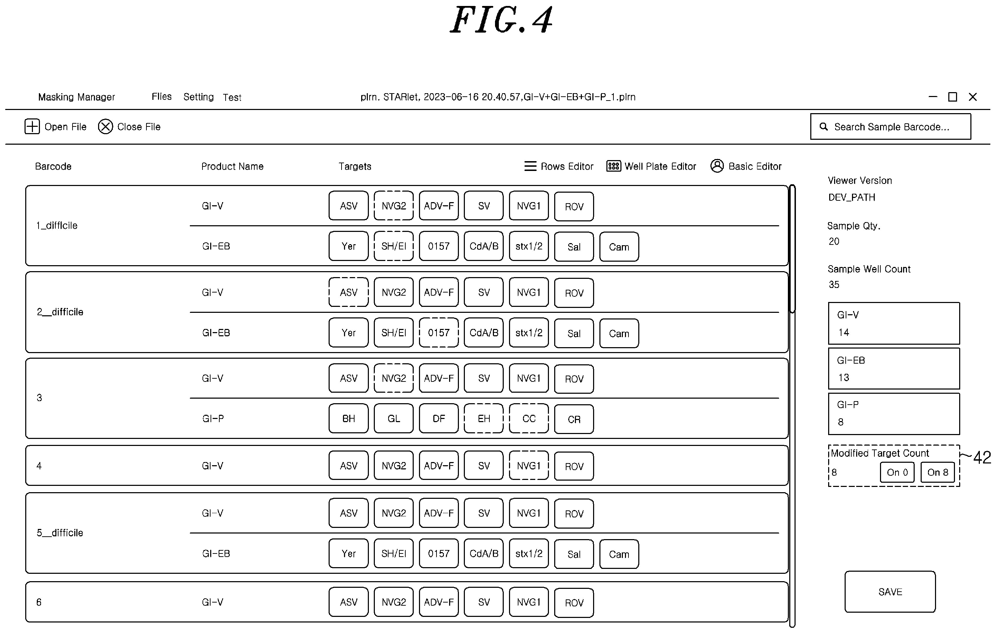

Therefore, the detection result display editing apparatus 100 may display identifiers of different analysis targets for each of the plurality of detection reagents as illustrated in FIG. 4 through the masking data input screen.

At this time, the detection result display editing apparatus 100 may selectively display only an analysis target of interest. For example, when an amplification reaction performs on a specimen of a patient named Kim using detection reagents A, B, C, and D, a detection result display target will be an analysis target set for each of the plurality of detection reagents, that is, an identifier for a plurality of biological target analytes.

However, although being an identifier of an analysis target set for each of the plurality of detection reagents, an identifier of some analysis targets may not be a target of interest.

Furthermore, when an amplification reaction performs on each specimen of patients named Kim, Lee, and Park using a detection reagent A, identifiers which are a plurality of analysis targets set in the detection reagent A for each patient are detection result display targets, but identifiers of targets of interest may be different from each other for each patient.

For this purpose, the present disclosure may receive editing information that determines whether to mask an amplification reaction result in the detection result display apparatus 300 for an identifier of at least one biological target analyte among the identifiers for the plurality of biological target analytes amplified in that reaction well through the masking data input screen of the detection result display editing apparatus 100.

According to the present disclosure, when identifiers for a plurality of biological target analytes set as analysis targets for each of the plurality of detection reagents displayed on the masking data input screen 40 are displayed, a user may selectively select an identifier not of interest for each of the plurality of detection reagents through a selection action such as clicking and check the identifier to display a masking intention for the corresponding identifier.

That is, regardless of a positive or negative reading, an identifier whose detection result is not desired to be checked may be masked by designating the identifier as an off-target.

In such a masking method, in one embodiment, a corresponding identifier displays in a specific color or a specific symbol, but is not limited thereto, and masking may be possible through various methods when distinguished from an identifier of a target of interest.

As illustrated in FIG. 4, according to the present disclosure, the detection result display editing apparatus 100 displays different identifiers for each of the plurality of detection reagents (Product Name in FIG. 4) as analysis targets through the masking data input screen 40, and a specific identifier selected by a user from the displayed identifiers (Target in FIG. 4) for each of the plurality of detection reagents may be displayed as an off-target using, for example, a gray color through a user selection.

For example, referring to FIG. 4, identifiers (ASV, NVG2, ADV-F, SV, NVG1, and ROV) for a plurality of biological target analytes capable of being detected in a sample barcoded as 1_difficile, that is, set in a GI-V detection reagent to check an infection of Gastrointestinal Tract Infections using the GI-V detection reagent are displayed on the masking data input screen 40. Among them, NVG2 is distinguished from ASV, ADV-F, SV, NVG1, and ROV, meaning an identifier whose detection result is not desired to be checked, and NVG2 is selected by a user and designated as an off-target to mask the corresponding identifier.

Furthermore, in identifiers (Yer, Sh/EI, O157, CdA/B, stx1/2, Sat, and Cam) for a plurality of biological target analytes capable of being detected using a GI-EB detection reagent in a sample barcoded as 2_difficile, Sh/EI is displayed as an identifier whose detection result is not desired to be checked by masking.

Meanwhile, in a sample barcoded as 5_difficile and accommodating GI-V and GI-EB detection reagents, since any target is not masked for an identifier, this unmasked state means to display detection results for all identifiers capable of being detected in a corresponding detection reagent.

As such, different identifiers for each of the plurality of detection reagents displayed through the masking data input screen 40 are naturally samples prepared in the nucleic acid preparation apparatus 200 and accommodated in a plate on which an amplification reaction is performed by a nucleic acid detection apparatus.

Depending on an embodiment, identifiers for each of the plurality of detection reagents may be displayed in a unit of a barcode on the masking data input screen 40. This is because a patient specimen is independently accommodated in a single reaction well, and thus the patient specimen accommodated together with the detection reagent in the reaction well may be barcoded as patient identification information and classified for each reaction well.

A barcode may be a patient identification code and/or a patient specimen code read through a barcode recognition apparatus. Furthermore, the barcode may represent contents capable of identifying between samples, such as a gender, age, and type of a biological sample, and classifying the sample based on necessary contents.

Accordingly, on the masking data input screen, an identifier of a detection reagent in which a virus desired to be checked by a corresponding patient is set as an analysis target is displayed based on a patient code recognized as a barcode, and among them, an identifier not of interest may be displayed masked.

However, such a masking data input screen is merely an implementation example according to the present disclosure and is not limited thereto.

Therefore, on the masking data input screen, the aforementioned identifier for each of the plurality of detection reagents may be displayed for each barcode in a unit of a barcode, or may be displayed for each of a plurality of reaction wells in a unit of a reaction well.

Meanwhile, according to the present disclosure, an identifier displayed on the masking data input screen 40 in the detection result display editing apparatus 100 may be received from the detection result display apparatus 300.

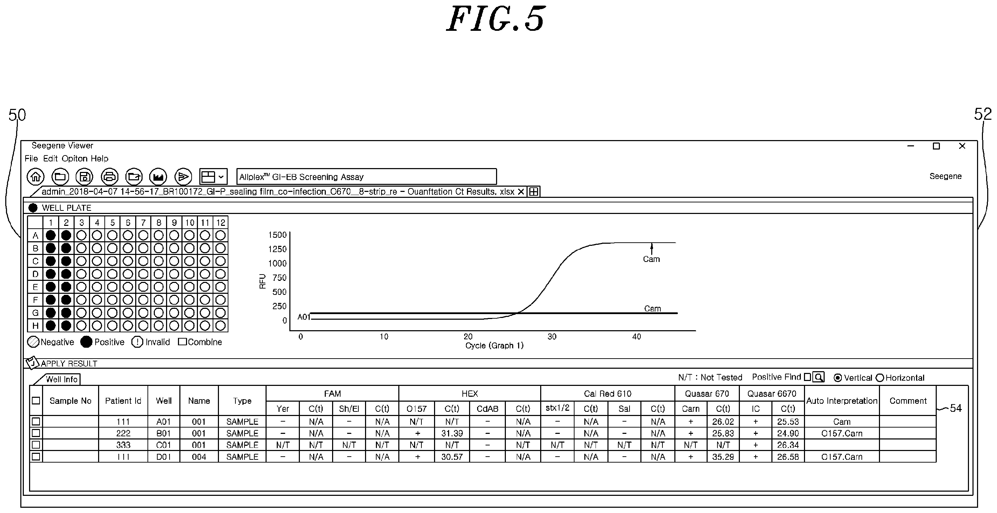

The detection result display apparatus 300 obtains an amplification reaction result, that is, a dataset, from the nucleic acid detection apparatus 500. As illustrated in FIG. 5, the detection result display apparatus 300 may generate an amplification curve 52 using a dataset. The detection result display apparatus 300 may remove noise from a dataset and generate the amplification curve 52 using the noise-removed dataset. The detection result display apparatus 300 may analyze data generated in a plate reaction well 50 and determine a positive/negative, a Ct (Cycle threshold) value, a signal value (e.g., an RFU value) at a specific cycle, and the like for each sample.

Removing noise from a dataset, generating an amplification curve, analyzing a dataset, determining a positive/negative, and the like may be performed by the nucleic acid detection apparatus 500.

Such a detection result display apparatus 300 has information on a plurality of biological target analytes capable of being amplified for each of the plurality of detection reagents, that is, an identifier.

For example, the detection reagent Allplex^{TM} Respiratory Panel1 for checking an infection of Respiratory Infections sets Influenza A virus (Flu A), Influenza A virus A-H1 (Flu A-H1), Influenza A virus A-H3 (Flu A-H3), Respiratory syncytial virus A (RSV A), Respiratory syncytial virus B (RSV B)... as amplifiable analysis targets. The detection reagent Allplex^{TM} GI-Virus Assay for checking an infection of Gastrointestinal Tract Infections sets Adenovirus, Astrovirus, and Norovirus as amplifiable analysis targets.

Therefore, the detection result display apparatus 300 obtains a dataset according to an amplification reaction result from the nucleic acid detection apparatus, and may display amplification data as illustrated in FIG. 5 based on the obtained dataset and a preset analysis target for each of the plurality of detection reagents.

FIG. 5 is a screen displaying an amplification reaction result for all target analytes before masking an identifier for a target analyte using the detection result display editing apparatus 100 for the present disclosure or before using the detection result display editing apparatus 100.

As illustrated in FIG. 5, the detection result display apparatus 300 may display an image of a plate 50 on which an amplification reaction performs, and display amplification data of a plurality of reaction wells included in the plate 50 as a graph 52 and/or a well information table 54. At this time, the amplification data may include at least one of a cycle number and a signal value set, a signal value at a specific cycle, a positive/negative determination result, and a Ct value.

On the table 54 displayed on the detection result display apparatus 300, amplification data including a name of a target gene to be detected in each channel (FAM, HEX, Cal Red 610, and Quasar 670), that is, an identifier for target analytes, a positive/negative determination result for each of a plurality of reaction wells (A01, B01, C01, D01, E01...), a Ct value, and the like may display.

For this purpose, the detection result display apparatus 300 previously stores information on a plurality of biological target analytes as an analysis target for each of the plurality of detection reagents, and when a dataset is obtained from the nucleic acid detection apparatus 500, analyzes the dataset to determine whether target analytes are present or absent in a sample, detects and stores data on the sample in a memory, and may display detection results such as a Ct value and a signal value (e.g., an RFU value) for each sample.

According to the present disclosure, the detection result display editing apparatus 100 receives an identifier from the detection result display apparatus 300, and identifies a detection reagent through plate setup information received from the nucleic acid preparation apparatus 200, so that an identifier for a plurality of biological target analytes capable of being detected for each of the plurality of detection reagents may display on the masking data input screen as illustrated in FIG. 4.

Alternatively, depending on an embodiment, the detection result display editing apparatus 100 may previously store an analysis target identifier for each of the plurality of detection reagents in the memory. In this case, when the detection result display editing apparatus 100 receives plate setup information from the nucleic acid preparation apparatus 200 and identifies detection reagent information for each of a plurality of reaction wells for a reaction plate on which an amplification reaction is to be performed, the detection result display editing apparatus 100 may display on the masking data input screen as illustrated in FIG. 4 using a previously stored identifier corresponding to the identified detection reagent.

And, depending on an embodiment, the detection result display editing apparatus 100 may receive an identifier from an external, for example, a medical server cloud 400, except for the aforementioned nucleic acid preparation apparatus 200 and/or detection result display apparatus 300 and/or nucleic acid detection apparatus.

Plate setup information may also be received from an external, for example, a connection apparatus, not from the nucleic acid preparation apparatus 200, and displayed on the masking data input screen 40 as illustrated in FIG. 4 by matching an identifier set in a detection reagent. In this case, a '.plrn' file as plate setup information generates in the connection apparatus.

Furthermore, depending on an embodiment, the detection result display editing apparatus 100 may receive all identifiers for each of the plurality of detection reagents displayed on the masking data input screen 40 from the outside at once, and depending on an embodiment, the detection result display editing apparatus 100 may display only an identifier for a plurality of biological target analytes on the masking data input screen 40.

In this way, an identifier displayed on the masking data input screen 40 is displayed to determine masking of the identifier when amplification data is displayed on the detection result display apparatus 300, and the detection result display editing apparatus 100 may display a plurality of biological target analytes capable of being detected based on a detection reagent included in a reaction well as an individually selectable object by a user using an identifier for each of the plurality of biological target analytes. Herein, the object may be in a selectable form such as a text and icons of various shapes, but is not limited thereto, and may be implemented in various forms when the object is an individually selectable object.

Therefore, an identifier displayed on the masking data input screen 40 may display by distinguishing an identifier selected for masking and an identifier not selected for masking according to masking of the identifier in the detection result display apparatus 300.

According to the present disclosure, masking of a corresponding identifier is determined in the detection result display apparatus 300 through an object selection, and when an object selection does not occur, a corresponding identifier may be displayed in the detection result display apparatus.

However, in selecting an identifier in the present disclosure, it will be obvious that a selection action is not particularly limited to masking and non-masking when not selected, and when a selection of an object distinguishes on the masking data input screen 40, masking of each object will be finally displayed as a result on the detection result display apparatus 300 according to a result of distinguishing.

In an embodiment, editing information may be generated through whether an object is selected, that is, an identifier for a plurality of biological target analytes for each of the plurality of detection reagents and the detection reagent obtained from accessed plate setup information.

On the masking data input screen 40 of the detection result display editing apparatus 100 according to the present disclosure, an identifier for each of the plurality of detection reagents included in a sample for each of a plurality of reaction wells for a reaction plate targeted for an amplification reaction displays during an amplification reaction start, during an amplification reaction, or after an amplification reaction completion in the nucleic acid detection apparatus 500 by setting an identifier for a plurality of biological target analytes capable of being amplified as an analysis target for each of the plurality of detection reagents. At this time, as described above, information of the identifier may be received from the detection result display apparatus 300 or received from other external apparatuses.

When a user selection occurs on identifiers displayed on the masking data input screen 40, an identifier on which the user selection occurs is masked when a detection result is displayed in the detection result display apparatus 300, and checking a detection result will be impossible.

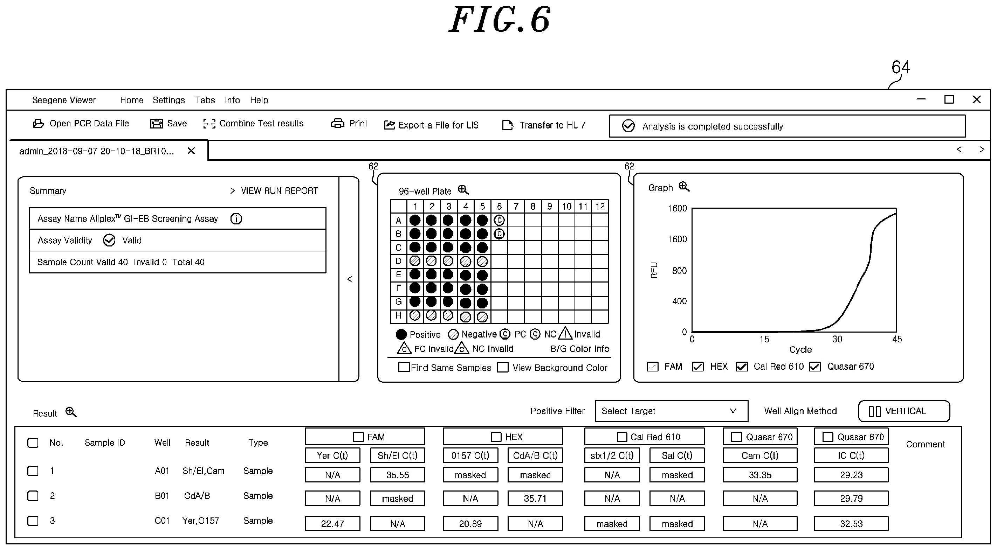

FIG. 6 illustrates an implementation example of a screen displaying an identifier masked in the detection result display editing apparatus 100 according to the present disclosure as amplification reaction result data in the detection result display apparatus 300. FIG. 6 displays amplification reaction result data by obtaining an amplification reaction result data set from the nucleic acid detection apparatus 500 by the detection result display apparatus 300, and using the obtained data set, and a reaction plate 60 on which an amplification reaction performs, an amplification reaction result graph 62 in each of a plurality of reaction wells, and a table 64 may display simultaneously on a single screen.

As illustrated in FIG. 6, in the detection result display apparatus 300, on the table 64 displaying amplification reaction result data, target analytes O157 and CdA/B for an HEX channel are masked in an A01 well, and a target analyte Sal for a Cal Red 610 channel is masked. In a C01 well, target analytes stx1/2 and Sal for a Cal Red 610 channel are masked.

As displayed on the plate 60 of FIG. 6, although the A01 well and the C01 well are samples on which an amplification reaction is performed, when detection results of some target analytes among a plurality of biological target analytes capable of being amplified by a detection reagent accommodated in each corresponding sample are not of interest, corresponding target analytes are displayed masked as on the table 64.

In addition, depending on an embodiment, information on a name of a detection reagent used in a target reaction plate on which amplification reaction result data displays (Assay Name in FIG. 6) and the number and validity of samples on which an amplification reaction performs in a reaction plate (Sample Count Valid in FIG. 6) may additionally display in the detection result display apparatus 300.

Target analytes displayed masked in the detection result display apparatus 300 as such follow plate setup information edited through the masking data input screen 40 in the detection result display editing apparatus 100 of the present disclosure.

Plate setup information transmitted from the detection result display editing apparatus 100 to the detection result display apparatus 300 may be in a form of a plrn file depending on an embodiment, and in this case, information on an identifier edited by masking records in a specific field of a plrn file. Specifically, a plrn file related to plate setup information records setup information for each of a plurality of reaction wells, an amplification reaction path, a plate type, and detection reagent information in each of various fields including a header, and particularly, detection reagent information may record in a Well Note field, and information on an identifier edited by masking may record in a Run Notes field. At this time, the detection result display editing apparatus 100 may convert masking of an identifier into a hexadecimal number and record a hexadecimal number in the Run Notes field. For example, when 6 identifiers edit, a binary number expressed as 10101100 converts into a hexadecimal number and records as AC, and when 14 identifiers edit, a binary number expressed as 1010110101011100 converts into a hexadecimal number and records as AD5C. In this case, even if the number of identifiers to be edited increases, a size of the Run Notes field may not be affected.

As described above, plate setup information receives from the nucleic acid preparation apparatus 200 according to the present disclosure, and when plate setup information receives, the detection result display editing apparatus 100 displays information on a corresponding detection reagent according to patient identification information for each barcode unit or for each of a plurality of reaction wells on the masking data input screen 40 based on received plate setup information, and matches identifier information for each of the plurality of detection reagents received from the detection result display apparatus 300 with data displayed on the masking data input screen 40 to display, thereby performing an editing determining masking of an identifier to be displayed as amplification reaction result data on the detection result display apparatus 300.

Herein, plate setup information received from the nucleic acid preparation apparatus 200 may be referred to as an original file, and in order to transmit information on an identifier masked through a masking data input screen in the detection result display editing apparatus 100 to the detection result display apparatus 100, an editing on the original file is typically performed.

Accordingly, a file different from the original file may be stored in the detection result display editing apparatus 100 through an editing storing editing information on which identifier is a masking target in a new file, not the original file. An edited file different from the original file is a file including masked identifier information, that is, Target on/off information, and is a file including new plate setup information different from the original file.

Therefore, in the detection result display editing apparatus 100, a file for plate setup information before editing and/or plate setup information after editing may be stored, and plate setup information for an original file may update or new plate setup information may be generated through an editing determining masking of a specific identifier.

Depending on an embodiment, each plate setup information in updating or generating may include at least one or more of a storage time, a storage date, an editor, detection reagent information, and a name of a nucleic acid preparation apparatus.

Such editing information may display 42 on the masking data input screen 40 by counting how many revisions occurred from the original file depending on an embodiment.

On the masking data input screen 40, an On number of identifiers in an On state basically displaying all identifiers for each of the plurality of detection reagents and an Off number of masked identifiers in the On state may be counted and displayed. In this case, a total sum of On/Off may be a total number of identifiers set for each of the plurality of detection reagents. This is to enable generation of re-editing information after generating editing information by counting the number of On identifiers and Off identifiers when re-editing information necessary for a second, third... or a second, third... generation file is required from an update or generation file for editing information generated by masking at least one specific identifier.

Each updated or generated file as such may be stored in a form of a template. Masking of an identifier for each of the plurality of detection reagents for each template may be checked according to a storage method, and such a template is to enable masking of a plurality of identifiers simultaneously at once through loading without individually selecting identifiers displayed on the masking data input screen 40.

Plate setup information further edited according to such a template may also be exported to the detection result display apparatus 300 and/or an external storage apparatus.

For example, even if a detection reagent is capable of amplifying all common causative bacteria for each age group, common causative bacteria of infectious diarrhea differ depending on age, so a result of presence or absence displays mainly on common causative bacteria for each age group, and when remaining causative bacteria store in a template form by masking, a corresponding template preferentially loads according to patient identification information obtained through a barcode to quickly check a result. Such a template may also be generated for each symptom.

A template may be generated by displaying mainly on identifiers preferentially considered for each symptom and masking remaining identifiers. In addition, a template may be generated by time. In a case of a short incubation period of 1 to 6 hours, a case of 8 to 16 hours, and a case of 16 to 72 hours, a template may be generated in advance mainly on suspected identifiers for each time period, and a result may be quickly checked by selectively loading a template according to time.

For example, time may be 1 hour, 2 hours, 3 hours, 4 hours, 5 hours, 6 hours, 7 hours, 8 hours, 9 hours, 10 hours or more, and may be 96 hours, 72 hours, 48 hours, 36 hours, 24 hours or less. In this way, a template may be obtained by presetting editing information and storing editing information in advance by time period, symptom, and age group. A symptom includes a type of a symptom, a mild degree, and a severe degree, and an age group may include all age groups from a newborn, an infant, a child, an adolescent, and middle-aged to an elderly person.

Meanwhile, in an embodiment, when the detection result display apparatus 300 displaying a specific identifier by masking exists in two or more versions, the detection result display editing apparatus 100 allows selecting a corresponding version.

For this purpose, the detection result display editing apparatus 100 allows selecting which detection result display apparatus to pair with before displaying the masking data input screen 40 depending on an embodiment, and accesses identifier information from a selected detection result display apparatus. An analysis target of a detection reagent may continuously update (add, delete), and since a detection result display apparatus may also continuously update due to limits on licensing, the detection result display editing apparatus 100 capable of performing masking by receiving identifier information depending on the detection result display apparatus depending on an embodiment may select a detection result display apparatus to be paired before performing masking.

FIG. 3 is a flowchart illustrating a method generating editing information determining whether to mask an amplification reaction result for an identifier of at least one biological target analyte among identifiers for a plurality of biological target analytes in the detection result display editing apparatus 100 according to the present disclosure.

The method according to the present disclosure is a computer-implemented method editing a display of detection results of a plurality of biological target analytes performed in a detection result display editing apparatus for a plurality of biological target analytes using a memory, a processor, and one or more programs stored in the memory and executable by the processor.

Referring to FIG. 3, in S310, the detection result display editing apparatus 100 accesses plate setup information for a reaction plate on which an amplification reaction is to be performed using a detection reagent for a multiplex nucleic acid amplification reaction capable of detecting the plurality of biological target analytes.

At this time, the plate setup information comprises setup information for each of a plurality of reaction wells, and the setup information for each of the plurality of reaction wells comprises identifiers for the plurality of biological target analytes amplified in that reaction well.

In S312, the identifiers are displayed on the masking data input screen of the detection result display editing apparatus 100.

In S314, editing information that determines whether to mask an amplification reaction result in the detection result display apparatus 300 for an identifier of at least one biological target analyte among the identifiers for the plurality of biological target analytes amplified in that reaction well is received through the masking data input screen.

In S316, the received plate setup information is edited according to the editing information.

In S318, the edited plate setup information is provided to the detection result display apparatus 300 and/or the outside.

Since operations performed in each operation illustrated in FIG. 3 are identical to functions of the detection result display editing apparatus 100 examined above, descriptions of FIGS. 1 and 2, and FIGS. 4 to 6 described above will be cited.

Combinations of blocks and respective operations of flowcharts attached to the present disclosure may be performed by a computer program.

When these computer programs are executed by one or more processors included in a detection result display editing apparatus, the one or more processors may cause the detection result display editing apparatus to perform accessing plate setup information for a reaction plate on which an amplification reaction is to be performed using a detection reagent for a multiplex nucleic acid amplification reaction capable of detecting the plurality of biological target analytes; displaying identifiers on a masking data input screen of the detection result display editing apparatus, wherein the plate setup information comprises setup information for each of a plurality of reaction wells, and the setup information for each of the plurality of reaction wells comprises identifiers for the plurality of biological target analytes amplified in that reaction well; receiving editing information that determines whether to mask an amplification reaction result in a detection result display apparatus for an identifier of at least one biological target analyte among the identifiers for the plurality of biological target analytes amplified in that reaction well through the masking data input screen; and editing the received plate setup information according to the editing information and providing the edited plate setup information. Such a program may be stored in a non-transitory computer-readable storage medium to implement a function by a specific method. Furthermore, unless clearly described to the contrary, terms such as "include", "comprise", or "have" described above mean a corresponding component may be inherent, and therefore, should be interpreted as further including another component rather than excluding the other component. All terms including technical or scientific terms have the same meaning as commonly understood by those of ordinary skill in the art to which the present disclosure pertains, unless defined otherwise. Terms commonly used such as terms defined in a dictionary should be interpreted as matching a meaning in the context of a related technology, and unless explicitly defined in the present disclosure, are not interpreted in an ideal or excessively formal meaning.

The above description is merely illustrative of a technical spirit of the present disclosure, and various modifications and variations will be possible to those of ordinary skill in the art to which the present disclosure pertains without departing from essential characteristics of the present disclosure. Therefore, the embodiments disclosed in the present disclosure are not intended to limit the technical spirit of the present disclosure but are for illustrative purposes, and the scope of the technical spirit of the present disclosure is not limited by these embodiments. The scope of protection of the present disclosure should be interpreted by the claims below, and all technical spirits within the equivalent scope thereof should be interpreted as being included in the scope of rights of the present disclosure.

As the specific parts of the present disclosure have been described in detail above, it is obvious to those of ordinary skill in the art such specific descriptions are merely preferred implementations, and the scope of the present disclosure is not limited thereto. Accordingly, the substantial scope of the present disclosure will be defined by the appended claims and equivalents thereof.

## Claims

1. A computer-implemented method for editing a display of detection results of a plurality of biological target analytes to be performed by a detection result display editing apparatus, the method comprising:
(a) accessing, by the detection result display editing apparatus, plate setup information for a reaction plate on which an amplification reaction is to be performed using a detection reagent for a multiplex nucleic acid amplification reaction capable of detecting the plurality of biological target analytes,
wherein the detection result display editing apparatus includes (i) a memory, (ii) a processor, and (iii) one or more programs stored in the memory and executable by the processor,
wherein the plate setup information comprises setup information for each of a plurality of reaction wells, and
wherein the setup information for each of the plurality of reaction wells comprises identifiers for the plurality of biological target analytes amplified in that reaction well;
(b) displaying the identifiers on a masking data input screen of the detection result display editing apparatus;
(c) receiving editing information that determines whether to mask an amplification reaction result in a detection result display apparatus for an identifier of at least one biological target analyte among the identifiers for the plurality of biological target analytes amplified in that reaction well through the masking data input screen; and
(d) editing the received plate setup information according to the editing information and providing the edited plate setup information.

2. The method of claim 1, wherein the accessing comprises accessing the plate setup information i) stored in the detection result display apparatus or an external apparatus or ii) stored in the detection result display editing apparatus, and wherein the plate setup information stored in the detection result display editing apparatus is received from the detection result display apparatus or the external apparatus.

3. The method of claim 1, wherein the identifiers are names of the plurality of biological target analytes to be amplified using the detection reagent, and wherein, when the detection reagent comprises a plurality of detection reagents, identifiers of biological target analytes for the plurality of detection reagents are displayed on the masking data input screen according to each of the plurality of detection reagents.

4. The method of claim 1, wherein the displaying comprises displaying the identifiers for the plurality of biological target analytes as a plurality of objects individually selectable by a user.

5. The method of claim 4, wherein the displaying comprises distinctively displaying at least one selected object and at least one unselected object among the plurality of objects displayed on the masking data input screen.

6. The method of claim 5, wherein, for the selected object, masking of the corresponding identifier and the amplification reaction result is determined in the detection result display apparatus, and wherein, for the unselected object, the corresponding identifier and the amplification reaction result are displayed in the detection result display apparatus.

7. The method of claim 4, wherein the editing information is generated using information indicating whether each of the individually selectable objects is selected based on the accessed plate setup information.

8. The method of claim 2, wherein the plate setup information stored in the detection result display editing apparatus is further edited according to a template in which identifiers to be masked among the plurality of biological target analytes capable of being detected are predefined based on the detection reagent.

9. The method of claim 8, wherein the template is preset editing information, and wherein whether the amplification reaction result is masked for identifiers of the plurality of biological target analytes is stored in the preset editing information.

10. The method of claim 8, wherein the template is classified based on at least one of time elapsed since symptom onset, a symptom, gender, and an age group.

11. The method of claim 10, wherein the time elapsed since symptom onset is 1 hour, 2 hours, 3 hours, 4 hours, 5 hours, 6 hours, 7 hours, 8 hours, 9 hours, 10 hours or more, or 96 hours, 72 hours, 48 hours, 36 hours, 24 hours or less.

12. The method of claim 10, wherein the symptom is any one of a symptom type and a symptom severity indicating whether the symptom is mild or severe.

13. The method of claim 10, wherein the gender includes female and male, and wherein the age group ranges from newborns to elderly persons.

14. The method of claim 2, wherein, when the plate setup information is stored in the detection result display apparatus or the external apparatus, the method further comprises exporting the plate setup information edited according to the editing information to the detection result display apparatus and/or an external storage apparatus.

15. The method of claim 8, wherein the method further comprises exporting the plate setup information further edited according to the template to the detection result display apparatus and/or an external storage apparatus.

16. The method of claim 14 or claim 15, wherein, in the exporting, the editing information indicating whether the identifiers for the plurality of biological target analytes are masked is converted into a hexadecimal number and exported.

17. A detection result display editing apparatus for editing a display of detection results of a plurality of biological target analytes, the detection result display editing apparatus comprising: a memory; a processor; and one or more programs stored in the memory and executable by the processor, wherein the one or more programs, when executed by the processor, cause the detection result display editing apparatus to:
access plate setup information for a reaction plate on which an amplification reaction is to be performed using a detection reagent for a multiplex nucleic acid amplification reaction capable of detecting the plurality of biological target analytes,
wherein the plate setup information comprises setup information for each of a plurality of reaction wells, and
wherein the setup information for each of the plurality of reaction wells comprises identifiers for the plurality of biological target analytes amplified in that reaction well;
display the identifiers on a masking data input screen of the detection result display editing apparatus;
receive editing information that determines whether to mask an amplification reaction result in a detection result display apparatus for an identifier of at least one biological target analyte among the identifiers for the plurality of biological target analytes amplified in that reaction well through the masking data input screen; and
edit the received plate setup information according to the editing information and provide the edited plate setup information.

18. A non-transitory computer-readable storage medium storing a computer program executable by one or more processors included in a detection result display editing apparatus, wherein the computer program, when executed by the one or more processors, causes the detection result display editing apparatus to perform a method comprising:
accessing, by the detection result display editing apparatus, plate setup information for a reaction plate on which an amplification reaction is to be performed using a detection reagent for a multiplex nucleic acid amplification reaction capable of detecting the plurality of biological target analytes,
wherein the plate setup information comprises setup information for each of a plurality of reaction wells, and
wherein the setup information for each of the plurality of reaction wells comprises identifiers for the plurality of biological target analytes amplified in that reaction well;
displaying the identifiers on a masking data input screen of the detection result display editing apparatus;
receiving editing information that determines whether to mask an amplification reaction result in a detection result display apparatus for an identifier of at least one biological target analyte among the identifiers for the plurality of biological target analytes amplified in that reaction well through the masking data input screen; and
editing the received plate setup information according to the editing information and providing the edited plate setup information.
